# EUROPÄISCHE PATENTSCHRIFT

(11) **EP 2 229 366 B1**
(45) Veröffentlichungstag und Bekanntmachung des Hinweises auf die Patenterteilung: **22.05.2013**
(21) Anmeldenummer: 08859028.6
(22) Anmeldetag: 08.12.2008
(51) Int. Cl.: C07D 233/58

(54) **Verfahren zur Aufarbeitung von Gemischen, welche Imidazoliumsalze enthalten**
Method for reprocessing mixtures containing imidazolium salts
Procédé pour traiter des mélanges contenant des sels d'imidazolium

(30) Priorität: 12.12.2007 EP 07122977
(43) Veröffentlichungstag der Anmeldung: 22.09.2010
(73) Patentinhaber: BASF SE, 67056 Ludwigshafen (DE)
(72) Erfinder: SIEMER, Michael, 68159 Mannheim (DE); MAASE, Matthias, Mendham New Jersey 07945 (US); RICHTER, Ingo, 68723 Schwetzingen (DE)
(86) Internationale Anmeldenummer: PCT/EP2008/067025
(87) Internationale Veröffentlichungsnummer: WO 2009/074538

(56) Entgegenhaltungen:
- EP-A- 1 470 846
- WO-A-91/14678
- WO-A-2005/019183
- WO-A-2005/021484
- DE-A1-102004 058 907

## Beschreibung

Die Erfindung betrifft ein Verfahren zur Aufhellung von Gemischen, welche Imidazoliumsalze enthalten, dadurch gekennzeichnet, dass die Gemische mit einem Oxidationsmittel behandelt werden.

Imidazoliumsalze haben eine große anwendungstechnische Bedeutung als ionische Flüssigkeiten. Unter dem Begriff ionische Flüssigkeiten werden Salze mit einem Schmelzpunkt kleiner 200°C, insbesondere bei Raumtemperatur flüssige Salze verstanden.

Ionische Flüssigkeiten, insbesondere Imidazoliumsalze, eignen sich z. B. als Lösemittel in viele technische Anwendungen, z. B. auch für das Lösen von Zellulose.

Gewünscht sind daher möglichst einfache und kostengünstige Verfahren zur Herstellung derartiger Imidazoliumsalze in möglichst hoher Reinheit und Qualität.

In WO 91/14678 wird ein einstufiges Verfahren zur Herstellung von Imidazoliumsalzen aus einer α -Dicarbonylverbindung, einem Aldehyd, einem Amin und einer Säure beschrieben (Arduengo-Verfahren)

Bei diesem Verfahren wird im Allgemeinen ein dunkel bis schwarz gefärbtes Gemisch erhalten, welches die gewünschten Imidazolium-salze enthält. Die Farbe des Gemisches ist offensichtlich auf einen Gehalt an Nebenprodukten zurückzuführen.

Ein mehrstufiges Verfahren wird z.B. in WO 2005/021484 beschrieben. Bei diesem Verfahren wird zunächst ein einfach substituiertes 1-Imidazol hergestellt. Dieses 1-Imidazol kann durch Destillation aus dem erhaltenen, dunkel gefärbten Gemisch abgetrennt werden. Erst danach erfolgt die Bildung des disubstituierten 1,3 Imidazoliumsalzes durch Umsetzung mit einem Dialkylcarbonat (Carbonat-Verfahren). Vorteilhaft wäre es, wenn auf den Verfahrensschritt der Abtrennung des 1-Imidazols verzichtet werden könnte.

Aufgabe der vorliegenden Erfindung ist daher ein möglichst einfaches Verfahren zur Verbesserung Herstellung von Gemischen, welche Imidazoliumsalze enthalten. Die Gemische sollen befriedigende optische und anwendungstechnische Eigenschaften haben und sollen möglichst ohne weitere Aufarbeitung, bzw. ohne Abtrennung der 1,3 disubstituierten Imidazole für technische Anwendungen, z. B. als Lösemittel für Zellulose geeignet sein.

Demgemäß wurde das eingangs definierte Verfahren gefunden.

### Zu den Gemischen

Vorzugsweise werden bei dem erfindungsgemäßen Verfahren Gemische verwendet, die bei der Herstellung von Imidazoliumsalzen aus Carbonylverbindungen und Aminen und gegebenenfalls weiteren Verbindungen erhalten werden. Insbesondere handelt es sich um Gemische, welche N,N-disubstituierte Imidazoliumsalze enthalten.

Es kann sich z. B. um Gemische handeln, die durch Umsetzung einer α -Dicarbonylverbindung, einem Aldehyd, mindestens einem Amin und einer Säure zum Imidazoliumsalz und gegebenenfalls anschließendem Austausch des Anions erhalten werden (Arduengo-Verfahren).

Es kann sich auch um Gemische handeln, die durch Umsetzung einer α -Dicarbonylverbindung, einem Aldehyd, einem Amin und Ammoniak zum einfach substituierten Imidazol, anschließende Umsetzung mit einem Dialkylcarbonat zum 1,3 disubstituierten Imidazoliumsalz und gegebenenfalls anschließendem Austausch des Anions erhalten werden (Carbonat-Verfahren). Im Allgemeinen wurde bisher das nach der ersten Stufe erhaltene einfach substituierte Imidazol aus dem gefärbten, Nebenprodukte enthaltenden Gemisch abgetrennt. Diese Abtrennung ist mit der erfindungsgemäßen Oxidation nicht mehr notwendig. Die erfindungsgemäße Oxidation kann nach der ersten Stufe mit dem Gemisch, welches das einfach substituierte Imidazol enthält oder auch nach der 2. Stufe mit dem Gemisch, welches das disubstituierte Imidazol enthält, durchgeführt werden.

Bei beiden vorstehenden Verfahren kann die Oxidation vor oder nach einem sich gegebenenfalls noch anschließenden Anionenaustausch erfolgen. Vorzugsweise erfolgt die Oxidation vor einem Anionenaustausch, falls ein solcher beabsichtigt ist.

Besonders bevorzugt werden Gemische verwendet, die 1,3 disubstituierte Imidazoliumsalze der Formel I enthalten 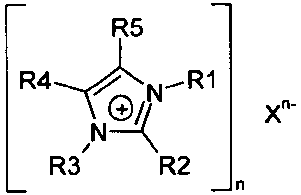 worin
R1 und R3 jeweils unabhängig voneinander für einen gleichen organischen Rest mit 1 bis 20 C-Atomen stehen
R2, R4,und R5 unabhängig voneinander für ein H-Atom oder für einen organischen Rest mit 1 bis 20 C-Atomen stehen
X für ein Anion und
n für 1, 2 oder 3 steht.

Derartige Gemische werden, wir oben ausgeführt, nach dem Arduengo-Verfahren oder nach der zweiten Stufe des Carbonat-Verfahrens erhalten.

Ebenfalls verwendet werden Gemische, welche einfach substituierte Imidazole der Formel II enthalten, 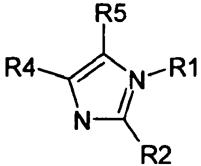 worin
R1 für einen organischen Rest mit 1 bis 20 C-Atomen steht,
R2, R4, und R5 unabhängig voneinander für ein H-Atom oder für einen organischen Rest mit 1 bis 20 C-Atomen stehen
und, wie oben ausgeführt, nach der ersten Stufe des Carbonat-Verfahrens erhalten werden.

Die nachfolgenden Ausführungen betreffen sowohl Verbindungen der Formel I als auch solche der Formel II, wobei alle Angaben zu R3, X und n sich nur auf Formel I beziehen.

R1 und R3 stehen vorzugsweise unabhängig voneinander für einen organischen Rest mit 1 bis 10 C-Atomen. Der organische Rest kann auch noch weitere Heteroatome, insbesondere Sauerstoffatome, zum Beispiel Hydroxylgruppen, Ethergruppen, Estergruppen oder Carbonylgruppen enthalten.

Insbesondere stehen R1 und R3 für einen Kohlenwasserstoffrest, der außer Kohlenstoff und Wasserstoff allenfalls noch Hydroxylgruppen, Ethergruppen, Estergruppen oder Carbonylgruppen enthalten kann.

R1 und R3 stehen besonders bevorzugt unabhängig voneinander für einen Kohlenwasserstoffrest mit 1 bis 20 C- Atomen, insbesondere mit 1 bis 10 C-Atomen, der keine sonstigen Heteroatome, z. B. Sauerstoff oder Stickstoff, enthält. Der Kohlenwasserstoffrest kann aliphatisch (wobei auch ungesättigte aliphatische Gruppen eingeschlossen sind) oder aromatisch sein oder sowohl aromatische als auch aliphatische Gruppen enthalten. Vorzugsweise stehen R1 und R2 für einen aliphatischen Kohlenwasserstoffrest.

Als Kohlenwasserstoffreste genannt seien z.B. die Phenylgruppe, Benzylgruppe, eine durch eine oder mehrere C1 bis C4 Alkylgruppen substituierte Phenylgruppe oder Benzylgruppe, Alkylgruppen und Alkenylgruppen, insbesondere die Allylgruppe.

Ganz besonders bevorzugt stehen R1 und R3 für eine C1 bis C10 Alkylgruppe. Als Alkylgruppe ist eine C1 bis C6 Alkylgruppe besonders bevorzugt, in einer besonderen Ausführungsform handelt es sich bei der Alkylgruppe um eine C1 bis C4 Alkylgruppe.

Ganz besonders bevorzugt stehen R1 und R3 unabhängig voneinander für eine Methyl-, Ethyl-, n-Propyl-, Isopropyl-, n-Butyl-, sec.Butyl oder tert.Butylgruppe, eine, wobei die Methyl-, Ethyl-, n-Propyl- und n-Butylgruppe besondere Bedeutung haben.

In einer bevorzugten Ausführungsform stehen R1 und R3 in Formel I jeweils für den gleichen organischen Rest, bei den Imidazoliumsalzen der Formel I handelt es sich daher insbesondere um symmetrische, disubstituierte Imidazoliumsalze.

In einer ebenfalls bevorzugten Ausführungsform handelt es sich bei den Imidazoliumsalzen der Formel I um Gemische von Imidazoliumsalzen mit unterschiedlichen Resten R1 und R3. Derartige Gemische sind erhältlich durch Verwendung von unterschiedlichen Aminen, z. bei primären Aminen mit unterschiedlichen Alkylgruppen. Das erhaltenen Gemisch enthält dann sowohl Imidazoliumsalze, in denen R1 und r3 identisch sind als auch Imidazolumsalze, in denen R1 und R3 unterschiedliche Bedeutung haben.

In einer besonderen Ausführungsform stehen in den Ammoniumsalzen der formel I
R1 und R3 für eine Methylgruppe,
R1 und R3 für eine Ethylgruppe,
R1 für eine Methylgruppe und R3 für eine Ethylgruppe
R1 für eine Methylgruppe und R3 für eine n Propylgruppe
R1 für eine Methylgruppe und R3 für eine n Butylgruppe
R1 für eine Methylgruppe und R3 für eine Allylgruppe
R1 für eine Ethylgruppe und R3 für eine Allylgruppe
R1 für eine Methylgruppe und R3 für eine Benzylgruppe
R1 für eine Ethylgruppe und R3 für eine Benzylgruppe

R2, R4, und R5 stehen unabhängig voneinander für ein H-Atom oder für einen organischen Rest mit 1 bis 20 C-Atomen stehen, wobei R4 und R5 auch zusammen einen aliphatischen oder aromatischen Ring ausbilden können. Der organische Rest kann neben Kohlenstoff und Wasserstoff auch Heteroatome wie Stickstoff oder Sauerstoff enthalten; vorzugsweise kann er Sauerstoff enthalten, insbesondere in Form von Hydroxylgruppen, Estergruppen, Ethergruppen oder Carbonylgruppen.

Insbesondere stehen R2, R4 und R5 unabhängig voneinander für ein H-Atom oder einen Kohlenwasserstoffrest, der außer Kohlenstoff und Wasserstoff allenfalls noch Hydroxylgruppen, Ethergruppen, Estergruppen oder Carbonylgruppen enthalten kann.

R2, R4 und R5 stehen bevorzugt unabhängig voneinander für ein Wasserstoffatom oder einen Kohlenwasserstoffrest mit 1 bis 20 C- Atomen, insbesondere mit 1 bis 10 C-Atomen, der keine sonstigen Heteroatome, z. B. Sauerstoff oder Stickstoff, enthält. Der Kohlenwasserstoffrest kann aliphatisch (wobei auch ungesättigte aliphatische Gruppen eingeschlossen sind) oder aromatisch sein oder sowohl aus aromatischen als auch aus aliphatischen Gruppen bestehen, wobei R4 und R5 auch auch einen aromatischen oder aliphatischen Kohlewasserstoffring ausbilden können, der gegebenenfalls durch weitere aromatische oder aliphatische Kohlenwasserstoffgruppen substituiert sein kann (die Anzahl der C-Atome des gegebenenfalls substituierten Kohlenwasserstoffrings einschließlich der Substituenten kann dabei vorzugsweise maximal 40, insbesondere maximal 20, besonders bevorzugt maximal 15 bzw. maximal 10 betragen). Als Kohlenwasserstoffreste genannt seien z.B. die Phenylgruppe, eine Benzylgruppe, eine durch eine oder mehrere C1 bis C4 Alkylgruppen substituierte Phenylgruppe oder Benzylgruppe, Alkylgruppen, Alkenylgruppen und, im Falle, dass R4 und R5 einen Ring ausbilden, ein durch R4 und R5 ausgebildeter aromatischer 5 - oder 6 Ring, ein Cyclohexen - oder Cyclpenten, wobei diese Ringsysteme insbesondere durch eine oder mehrere C1 bis C10, insbesondere C1 bis C4 Alkylgruppen substituiert sein können.
Als Kohlenwasserstoffrest sind aliphatische Kohlenwasserstoffreste bevorzugt.

Besonders bevorzugt stehen R2, R4 und R5 unabhängig voneinander für ein H-Atom, eine C1 bis C8 Alkylgruppe oder eine C1-C8 Alkenylgruppe, z. B. eine Allylgruppe,
Ganz besonders bevorzugt stehen R2, R4 und R5 unabhängig voneinander für ein H-Atom, eine Methyl-, Ethyl-, n Propyl-, Isopropyl-, n Butyl-, sec.Butyl oder tert.Butylgruppe, wobei die Methyl-, Ethyl-, n Propyl- und n Butylgruppe besondere Bedeutung haben.

In einer besonderen Ausführungsform steht R2 unabhängig von den anderen Resten R 4 und R5 und den übrigen Resten R1 und R3 für ein H-Atom. Imidazoliumsalze der Formel I, in denen R2 für ein H Atom steht, sind im Rahmen der vorliegenden Erfindung besonders vorteilhaft, sie haben eine gute Löslichkeit in den Epoxyverbindungen und eine hohe Wirksamkeit als latenter Katalysator. In einer besonderen Ausführungsform steht R2 für ein H-Atom, wenn es sich bei dem Anion um ein Acetat handelt.

In einer besonderen Ausführungsform stehen
R2, R4 und R5 für ein H-Atom,
R2 für ein H-Atom oder eine C1 bis C4 Alkylgruppe und R4, R5 für ein H-Atom oder eine C1 bis C4 Alkylgruppe

Als Einzelfälle für die Kationen der Verbindungen der Formel I seien genannt:
1-Butyl-3-methyl-imidazolium (R1= Butyl, R3 = Methyl)
1-Butyl-3-ethyl-imidazolium (R1=Butyl, R3=Ethyl)
1,3-Di-methyl-imidazolium (R1= Methyl, R3 =Methyl)
1,3-Di-ethyl-imidazolium (R1=Ethyl, R3=Ethyl)
1-Ethyl-3-methyl-imidazolium (R1=Ethyl, R3 =Methyl)
1-Ethyl-2,3 dimethyl- imidazolium (R1 =Ethyl, R2=Methyl, R3=Methyl)

Entsprechend seien als Imidazolverbindungen der Formel II genannt:
1-Methyl-imidazol
1-Ethyl-imidazol
1-Propyl-imidazol
1-Butyl-imidazol

In Formel I steht n für 1, 2 oder 3; das Anion hat entsprechend ein, zwei oder drei negative Ladungen und entsprechend liegen im Salz ein, zwei oder drei Imidazoliumkationen vor.

Bevorzugt steht n für 1 oder 2, besonders bevorzugt steht n für 1; das Anion ist daher besonders bevorzugt einwertig.

In Formel I steht X vorzugsweise für das Anion einer Wasserstoffsäure.

Als geeignete Anionen X⁻ seien z. B. Verbindungen mit einer oder mehreren Carboxylatgruppen (kurz Carboxylate) genannt.

Als derartige Carboxylate seien insbesondere organische Verbindungen mit 1 bis 20 C-Atomen genannt, die ein oder zwei, vorzugsweise eine Carboxylatgruppe enthalten. Es kann sich dabei sowohl um aliphatische als auch um aromatische Verbindungen handeln, wobei unter den aromatischen Verbindungen solche verstanden werden, die aromatische Gruppen enthalten. Besonders bevorzugt sind aliphatische oder aromatische Verbindungen, die außer den Sauerstoffatomen der Carboxylatgruppe keine weiteren Heteroatome enthalten oder allenfalls noch ein oder zwei Hydroxylgruppen, Carbonylgruppen oder Ethergrupen enthalten. Ganz besonders bevorzugt sind aliphatische oder aromatische Verbindungen, die außer den Sauerstoffatomen der Carboxylatgruppe keine weiteren Heteroatome enthalten.

Als Verbindungen mit zwei Carboxylatgruppen seien z.B. die Anionen der der Phthalsäure, der Isophthalsäure, der C2 bis C6 Dicarbonsäuren, z.B. Oxalsäure Malonsäure, Bernsteinsäure, Glutarsäure, Adipinsäure genannt.

Als Verbindungen mit einer Carboxylatgruppe seien die Anionen von aromatischen, aliphatischen, gesättigten oder ungesättigten C1 bis C20 Carbonsäuren, insbesondere Alkancarbonsäuren, Alkencarbonsäuren, Alkincarbonsäuren, Alkadiencarbonsäuren, Alkatriencarbonsäuren, Hydroxycarbonsäuren oder Ketocarbonsäuren aufgeführt. Geeignete Alkancarbonsäuren, Alkencarbonsäuren und Alkadiencarbonsäuren sind auch als Fettsäuren bekannt.

Genannt seien insbesondere die Anionen der Ameisensäure(Formiat) oder der Essigsäure (Acetate). Ganz besonders bevorzugtes Anion X ist das Acetat - Anion.

Weitere Anionen, welche insbesondere bei der Arduengo-Methode durch Wahl der entsprechende Säure als Ausgangsverbindung von Bedeutung sind, sind z. B. Halogenide, insbesondere Chlorid, Borate, Alkylsulfonate, insbesondere Methylsulfonat und Thiocyanat (Rhodanid).

Die Herstellung der Gemische aus den entsprechenden Ausgangsverbindungen (α - Dicarbonylverbindung, Aldehyd, ein Amin und die Wasserstoffsäure des Anions X) ist bekannt und erfolgt z. B. gemäß WO 91/14678 (Arduengo-Methode) oder WO 2005/021484 (Carbonat-Methode). Bei der α -Dicarbonylverbindung handelt es sich vorzugsweise um eine Verbindung der Formel

R4-CO-CO-R5 ,

wobei R4 und R5 die oben genannte Bedeutung haben.

Besonders bevorzugt handelt es sich um Glyoxal.

Bei dem Aldehyd handelt es sich insbesondere um ein Aldehyd der Formel R2-CHO, wobei R2 die oben genannte Bedeutung hat. Besonders bevorzugt ist Formaldehyd; der Formaldehyd kann auch in Form von Formaldehyd freisetzenden Verbindungen wie Paraformaldehyd oder Trioxan eingesetzt werden.

Bei den Aminen handelt es sich insbesondere um primäre Amine des Typs R- NH2. Der Rest R entspricht den Resten R1 und R3 der erhaltenen Imidazoliumsalze. Wenn ein definiertes primäres Amin eingesetzt wird, erhält man ein Imidazoliumsalz, in dem die Reste R1 und R3 identisch sind. Verwendet man ein Gemisch von Aminen (z. B. Gemisch aus R'-NH2 und R"-NH2) so erhält man ein Gemisch von Imidazoiliumsalzen (Gemisch von Salzen mit R1 und R3 = R', R1 und R3 = R" und Salzen mit R1 = R' und R3 = R").

Bei der Wasserstoffsäure handelt es sich um die gewünschte Wasserstoffsäure des Anions X, vorzugsweise um eine Alkancarbonsäure, besonders bevorzugt um Essigsäure.
Weitere Bestandteile der Gemische

Die Gemische können Lösemittel enthalten. Soweit lösemittel enthalten sind, handelt es sich vorzugsweise um Wasser, ein mit Wasser mischbares Lösemittel oder deren Gemische. Als mit Wasser mischbare Lösemittel seien insbesondere protische Lösemittel, vorzugsweise aliphatische Alkohole oder Ether mit maximal 4 Kohlenstoffatomen, z. B. Methanol, Ethanol, Methylethylether, Tetrahydrofuran genannt. Geeignete protische Lösemittel sind mit Wasser in jedem Verhältnis mischbar (bei 1 bar, 21 °C). Besonders bevorzugtes Lösemittel ist Wasser.

Das erfindungsgemäße Verfahren kann mit Lösemittel-haltigen Gemischen oder auch mit Lösemittel-freien Gemischen durchgeführt werden.

Die Gemische haben eine dunkle Färbung, diese Färbung ist auf Nebenprodukte zurückzuführen, die bei dem Herstellungsverfahren für die Imidazoliumsalze bzw. Imidazole (Arduengo- oder Carbonatmethode) entstehen.

### Zur Oxidation

Erfindungsgemäß werden die Gemische mit einem Oxidationsmittel behandelt.

Geeignete Oxidationsmittel sind dem Fachmann bekannt. Oxydationsmittel sind Verbindungen mit hoher Elektronenaffinität (Elektrophilie). Stark elektrophile und damit als Oxydationsmittel geeignet Verbindungen sind z. B. Sauerstoff und Sauerstoff enthaltende Per-Verbindungen, insbesondere Wasserstoffperoxid, Metallperoxide oder organische Peroxide wie Natriumpersulfat oder tertiär Butylhydroperoxid, anorganische und organische Persäuren, z.B. Perjodsäure oder Percarbonsäuren, aber auch andere Verbindungen wie Schwefel oder Metallverbindungen hoher Wertigkeitsstufen (z.B. Eisen -III -verbindungen, Mangandioxid, Kaliumpermanganat, Chromsäure, Chromanhydrid, Bleidioxid oder Bleitetraacetat).

Bei dem Oxidationsmittel handelt es sich bevorzugt um Sauerstoff, Peroxide oder Persäuren, besonders bevorzugt ist Wasserstoffperoxid.

Das Oxidationsmittel kann z. B. gasförmig oder flüssig sein. In Betracht kommt insbesondere gasförmiger Sauerstoff, der in geeigneter Weise, z. B. durch Druck und/oder Einleiten unterhalb der Flüssigkeitsoberfläche, mit dem Reaktionsgemisch in Kontakt gebracht wird.

In Betracht kommen insbesondere auch flüssige Oxidationsmittel, insbesondere Oxidationsmittel, welche in geeigneten, mit dem Reaktionsgemisch mischbaren Lösmitteln gelöst sind. Als Lösemittel kommt insbesondere Wasser, einem mit Wasser mischbaren Lösemittel oder deren Gemische in Betracht, entsprechend den obigen Lösemitteln für das Gemisch.

Bevorzugt sind gasförmiger Sauerstoff und insbesondere Wasserstoffperoxid, vorzugsweise in Form obiger Lösungen, insbesondere als 10 bis 40 Gew. % ige Lösung.

Die Menge des Oxidationsmittels wird nach Bedarf gewählt; auf 1 Mol Imidazoliumsalz, bzw Imidazol (wobei die theoretisch aus dem Reaktionsansatz erhaltenen Menge zugrunde gelegt wird) können z. B. mindestens 0,01 bzw. mindestens 0,1 vorzugsweise mindestens 0,5 Mol Oxidationsmittel eingesetzt werden; die Menge kann insgesamt z. B. 0,01 bis 20 Mol, vorzugsweise 0,1 bis 20 Mol bzw. 0,5 bis 10 Mol Oxidationsmittel betragen.

Die Oxidation kann z.B. bei Temperaturen von 20 bis 100 °C, insbesondere 50 bis 90 °C bei Normaldruck durchgeführt werden bis eine Aufhellung des Ansatzes eintritt.

Vor oder nach der Oxidation kann eine Aufarbeitung oder teilweise Aufarbeitung des Reaktionsgemisches erfolgen. Insbesondere wird die weitere Aufarbeitung, soweit gewünscht, z. B. Abtrennung des Lösemittels erst nach der Oxidation vorgenommen.

Die erhaltenen Gemische haben eine deutlich geringere Färbung als bisher üblich. Nicht nahe liegender Weise verändert die Oxidation die Beschaffenheit der Nebenprodukte derart, dass die Verfärbung deutlich abnimmt. Bei der Herstellung nach der Carbonat-Methode ist eine Abtrennung des Imidazols (und damit einhergehender Reinigung) nach der ersten Stufe nicht mehr notwendig. Die erhaltenen Gemische eignen sich unmittelbar für die weiteren Verwendungen, z. B. als Lösemittel, insbesondere als Lösemittel für Cellulose.

### Beispiele

### 1. Beispiel

### Herstellung von 1,3-Diethylimidazolium-Acetat (EEIM-Acetat)

Reaktionsgleichung: 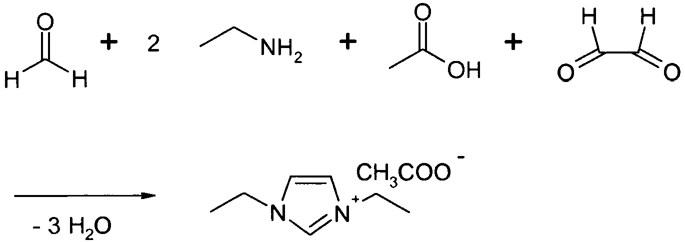 Rührgeschwindigkeiten: Reaktionsbehälter: 350 U/min

### Apparatur: 6l Vierhalskolben, Teflon-Halbmondrührer, Thermometer, Kühler, Tropftrichter

Ansatz:

| | | | | Substanz | |
|---|---|---|---|---|---|
| 252,1 | g | 8,15 | mol | Paraformaldehyd 97%ig | und |
| 325 | g | 18,04 | mol | Wasser | vorlegen |
| 1048,7 | g | 16,27 | mol | Ethylamin 70%ig in Wasser | zutropfen |
| 488,9 | g | 8,15 | mol | Eisessig | zutropfen |
| 1181,4 | g | 8,15 | mol | Glyoxal 40%ig in Wasser | zutropfen |

| Zeit | Temperatur | |
|---|---|---|
| 8:55 | 10°C | Paraformaldehyd und Wasser vorlegen, weiße Suspension, Ethylamin bei 20-30°C zutropfen, exotherme Reaktion, Eisbadkühlung |
| 9:35 | 23°C | Nach Zutropfen der Hälfte des Amins lässt die Exothermie nach, es entsteht eine klare Lösung |
| 9:50 | 23°C | Zulaufende, 30min bei RT nachrühren lassen |
| 10:35 | 25°C | Eisessig bei 20-30°C zutropfen, exotherme Reaktion, Eisbadkühlung, weißer Nebel entsteht |
| 11:10 | 25°C | Zulaufende, zwei klare Phasen, 20min bei RT nachrühren lassen |
| 11:30 | 21°C | Glyoxal bei 20-35°C zutropfen, der Ansatz wird einphasig und verfärbt sich von gelb bis schwarzbraun |
| 12:00 | 22°C | Zulaufende, über Nacht bei RT nachrühren lassen |

### Aufarbeitung:

Die erhaltene, schwarzbraune Produktmischung wurde auf 70°C erhitzen (pH 6,68) 1,5kg Wasserstoffperoxid 30%ig in ca. 1 h bei 70-80°C wurden zugetropft. Nach Zulaufende war eine Aufhellung festzustellen (pH 6,33), Es wurde noch 4h bei 80°C nachgerührt (keine Gasentwicklung), es trat eine weitere Aufhellung (hellorange (pH 6,08) ein.

Die Rührgeschwindigkeit wurde auf 480 U/min erhöht und ca. 375g NaOH 40%ig zur Neutralisierung von überschüssiger Säure in ca. 1,5h zugetropft. Die Temperatur hielt sich ohne weiteres Heizen bei 65°C, der pH steigt auf 9,5 bei sehr starker Gasentwicklung (Zersetzung von Wasserstoffperoxid, H2O2). Die Temperatur stieg auf 95°C, bei pH 10,3. Der Zulauf wurde gestoppt und über Nacht bei Raumtemperatur (RT) nachgerührt (Aufhellung bis gelb).

Das Produktgemisch (pH 11,2) wurde am Rotationsverdampfer eingeengt, 1,5kg Acetonitril zugegeben und über Nacht gerührt. Das Natriumsalz der überschüssigen Säure fällt aus und wird abgetrennt. Danach wurde das Gemisch am Rotationsverdampfer eingeengt.

Insgesamt wurden 1444,14 Gramm Produkt (EEIM-acetat, 1 H-NMR) erhalten.

### Beispiele 2 bis 12

Verschieden ionische Flüssigkeiten wurden 72 Stunden bei 110 °C im Trockenschrank gelagert und anschließend mit 0,5 Gramm Wasserstoffperoxid (H2O2) versetzt und danach visuell beurteilt und die Farbzahl nach Gardner bestimmt (je höher die Farbzahl desto dunkler).

| Imidazoliumsalz | Farbzahl Gardner vor H2O2 Zugabe | Visuelle Beurteilung nach Zugabe | Farbzahl nach Zugabe | H2O2 Gehalt |
|---|---|---|---|---|
| HMIM-HSO4 | | heller | | 0,09 Gew. % |
| EMIM-EtOSO3 | | unverändert | | |
| EMIM-HSO4 | 11,1 | heller | 10,1 | 0,83 Gew. % |
| EMIM-MeSO3 | 11,4 | heller | 5,1 | 0,11 Gew % |
| EMIM-SCN | 14,4 | | 14,6 | 59 ppm |
| BMIM-Acetat | 13,9 | heller | 8,3 | 0,5 % Gew % |
| BMIM-chlorid | | unverändert | | |
| BMIM-H2SO4 | 5,6 | heller | 4,4 | 0,79 Gew. % |
| BMIM-MeSO3 | 8,9 | heller | 6,2 | 0,48 Gew. % |
| BMIM-MeOSO3 | | unverändert | | |
| BMIM-SCN | 8,4 | trüb | 15,2 | 50 ppm |

HMIM, EMIM, BMIM bezeichnen Imidazoliumsalze der obigen Formel I mit Resten R1 und R3 gleich:
HMIM: R1 = H und R3 = Methyl
EMIM: R1 = Ethyl und R3 = Methyl
BMIM: R1 = Butyl und R3 = Methyl

## Patentansprüche

1. Verfahren zur Aufhellung von Gemischen, welche Imidazoliumsalze enthalten, **dadurch gekennzeichnet, dass** die Gemische mit einem Oxidationsmittel behandelt werden.

2. Verfahren gemäß Anspruch 1, **dadurch gekennzeichnet, dass** es sich um Gemische handelt, die bei der Herstellung von Imidazoliumsalzen aus Carbonylverbindungen und Aminen und gegebenenfalls weiteren Verbindungen erhalten werden.

3. Verfahren gemäß Anspruch 1 oder 2, **dadurch gekennzeichnet, dass** es sich um Gemische handelt, die durch Umsetzung einer α -Dicarbonylverbindung, einem Aldehyd, mindestens einem Amin und einer Säure zum Imidazoliumsalz und gegebenenfalls anschließendem Austausch des Anions erhalten erhalten werden (Arduengo-Verfahren zur Herstellung von Imidazoliumsalzen).

4. Verfahren gemäß einem der Ansprüche 1 bis 3, **dadurch gekennzeichnet, dass** es sich um Gemische handelt, die durch Umsetzung einer α -Dicarbonylverbindung, einem Aldehyd, einem Amin und Ammoniak zum einfach substituierten Imidazol (1.Stufe) anschließende Umsetzung mit einem Dialkylcarbonat zum 1,3 disubstituierten Imidazoliumsalz (2.Stufe) und gegebenenfalls anschließendem Austausch des Anions erhalten werden (Carbonat-Verfahren zur Herstellung von Imidazoliumsalzen), wobei die Oxidation mit dem nach der ersten Stufe erhaltenen Gemisch oder mit dem nach der zweiten Stufe erhaltenen Gemisch durchgeführt wird.

5. Verfahren gemäß einem der Ansprüche 1 bis 4, **dadurch gekennzeichnet, dass** die Gemische 1,3 disubstituierte Imidazoliumsalze der Formel I 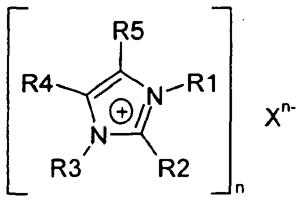 worin
R1 und R3 jeweils unabhängig voneinander für einen organischen Rest mit 1 bis 20 C-Atomen stehen,
R2, R4, und R5 unabhängig voneinander für ein H-Atom oder für einen organischen Rest mit 1 bis 20 C-Atomen stehen,
X für ein Anion steht und
n für 1, 2 oder 3 steht, enthalten.

6. Verfahren gemäß Anspruch 4, **dadurch gekennzeichnet, dass** die Gemische der 1. Stufe
einfach substituierte Imidazole der Formel II 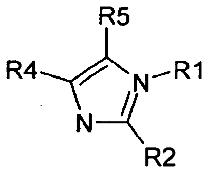 worin
R1 für einen organischen Rest mit 1 bis 20 C-Atomen steht.,
R2, R4, und R5 unabhängig voneinander für ein H-Atom oder für einen organischen Rest mit 1 bis 20 C-Atomen stehen, enthalten.

7. Verfahren gemäß einem der Ansprüche 1 bis 6, **dadurch gekennzeichnet, dass** R1 in Formel I und II und R3 in Formel I unabhängig voneinander für eine C1 bis C10 Alkylgruppe stehen.

8. Verfahren gemäß einem der Ansprüche 1 bis 7, **dadurch gekennzeichnet, dass** R2, R4 und R5 unabhängig voneinander für ein H-Atom oder eine C1 bis C10 Alkylgruppe stehen.

9. Verfahren gemäß einem der Ansprüche 1 bis 8, **dadurch gekennzeichnet, dass** X für das Anion einer Verbindung mit mindestens einer Carboxylatgruppe steht .

10. Verfahren gemäß einem der Ansprüche 1 bis 9, **dadurch gekennzeichnet, dass** X für das Acetat- Anion steht.

11. Verfahren gemäß einem der Ansprüche 1 bis 10, **dadurch gekennzeichnet, dass** die Gemische Wasser, ein Wasser mischbares Lösemittel oder deren Gemische als Lösemittel enthalten.

12. Verfahren gemäß einem der Ansprüche 1 bis 11, **dadurch gekennzeichnet, dass** es sich bei dem Oxidationsmittel um Sauerstoff, Peroxide oder Persäuren handelt.

13. Verfahren gemäß einem der Ansprüche 1 bis 12, **dadurch gekennzeichnet, dass** es sich bei dem Oxidationsmittel um Wasserstoffperoxid handelt.

14. Verfahren gemäß einem der Ansprüche 1 bis 13, **dadurch gekennzeichnet, dass** die Oxidation bei 20 bis 10 °C durchgeführt wird.

15. Verfahren gemäß einem der Ansprüche 1 bis 14, **dadurch gekennzeichnet, dass** das Oxidationsmittel vor der Entfernung des Lösemittels zugesetzt wird.

16. Gemische, erhältlich durch ein Verfahren gemäß einem der Ansprüche 1 bis 15.

17. Verwendung der Gemische gemäß Anspruch 16 als Lösemittel.

18. Verwendung der Gemische gemäß Anspruch 16 als Lösemittel für Zellulose.

## Claims

1. A method of lightening the color of mixtures comprising imidazolium salts, wherein the mixtures are treated with an oxidant.

2. The method according to claim 1, wherein the mixtures are mixtures which are obtained in the preparation of imidazolium salts from carbonyl compounds and amines and optionally further compounds.

3. The method according to claim 1 or 2, wherein the mixtures are mixtures which are obtained by reaction of an α-dicarbonyl compound, an aldehyde, at least one amine and an acid to form the imidazolium salt and optionally subsequent replacement of the anion (Arduengo process for preparing imidazolium salts).

4. The method according to any of claims 1 to 3, wherein the mixtures are mixtures which are obtained by reaction of an α-dicarbonyl compound, an aldehyde, an amine and ammonia to form the monosubstituted imidazole (1st step), subsequent reaction with a dialkyl carbonate to form the 1,3-disubstituted imidazolium salt (2nd step) and optionally subsequent replacement of the anion (carbonate process for preparing imidazolium salts), with the oxidation being carried out using the mixture obtained after the first step or using the mixture obtained after the second step.

5. The method according to any of claims 1 to 4, wherein the mixtures comprise 1,3-disubstituted imidazolium salts of the formula I 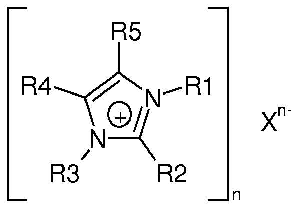 where
R1 and R3 are each, independently of one another, an organic radical having from 1 to 20 carbon atoms,
R2, R4 and R5 are each, independently of one another, an H atom or an organic radical having from 1 to 20 carbon atoms,
X is an anion and
n is 1, 2 or 3.

6. The method according to claim 4, wherein the mixtures of the 1st step comprise
monosubstituted imidazoles of the formula II 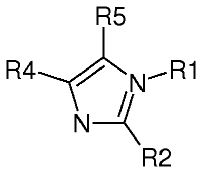 where
R1 is an organic radical having from 1 to 20 carbon atoms,
R2, R4 and R5 are each, independently of one another, an H atom or an organic radical having from 1 to 20 carbon atoms.

7. The method according to any of claims 1 to 6, wherein R1 in formulae I and II and R3 in formula I are each, independently of one another, a C1-C10-alkyl group.

8. The method according to any of claims 1 to 7, wherein R2, R4 and R5 are each, independently of one another, an H atom or a C1-C10-alkyl group.

9. The method according to any of claims 1 to 8, wherein X is the anion of a compound having at least one carboxylate group.

10. The method according to any of claims 1 to 9, wherein X is the acetate anion.

11. The method according to any of claims 1 to 10, wherein the mixtures comprise water, a solvent which is miscible in water or a mixture thereof as solvent.

12. The method according to any of claims 1 to 11, wherein the oxidant is oxygen, a peroxide or a peracid.

13. The method according to any of claims 1 to 12, wherein the oxidant is hydrogen peroxide.

14. The method according to any of claims 1 to 13, wherein the oxidation is carried out at from 20 to 10°C.

15. The method according to any of claims 1 to 14, wherein the oxidant is added before removal of the solvent.

16. A mixture which can be obtained by a method according to any of claims 1 to 15.

17. The use of the mixture according to claim 16 as solvent.

18. The use of the mixture according to claim 16 as solvent for cellulose.

## Revendications

1. Procédé pour éclaircir des mélanges qui contiennent des sels d'imidazolium, **caractérisé en ce que** les mélanges sont traités avec un oxydant.

2. Procédé selon la revendication 1, **caractérisé en ce qu'**il s'agit de mélanges qui sont obtenus lors de la préparation de sels d'imidazolium à partir de composés carbonyle et d'amines et le cas échéant d'autres composés.

3. Procédé selon la revendication 1 ou 2, **caractérisé en ce qu'**il s'agit de mélanges qui sont obtenus par transformation d'un composé α-dicarbonyle, d'un aldéhyde, d'au moins une amine et d'un acide en sel d'imidazolium et le cas échéant par remplacement consécutif de l'anion (procédé Arduengo pour la préparation de sels d'imidazolium).

4. Procédé selon l'une quelconque des revendications 1 à 3, **caractérisé en ce qu'**il s'agit de mélanges qui sont obtenus par transformation d'un composé α-dicarbonyle, d'un aldéhyde, d'une amine et d'ammoniaque en un imidazole monosubstitué (1ère étape), transformation consécutive avec un dialkylcarbonate en sel d'imidazolium 1,3-disubstitué (2ème étape) et le cas échéant remplacement consécutif de l'anion (procédé carbonate pour la préparation de sels d'imidazolium), l'oxydation étant réalisée sur le mélange obtenu après la première étape ou sur le mélange obtenu après la deuxième étape.

5. Procédé selon l'une quelconque des revendications 1 à 4, **caractérisé en ce que** les mélanges contiennent des sels d'imidazolium 1,3-disubstitués de formule I 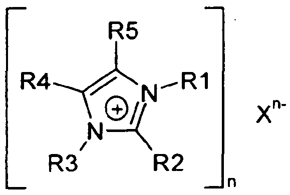 où
R1 et R3 représentent, à chaque fois indépendamment l'un de l'autre, un radical organique comprenant 1 à 20 atomes de carbone,
R2, R4, et R5 représentent, indépendamment l'un de l'autre, un atome d'hydrogène ou un radical organique comprenant 1 à 20 atomes de carbone
X représente un anion et
n vaut 1, 2 ou 3.

6. Procédé selon la revendication 4, **caractérisé en ce que** les mélanges de la 1ère étape contiennent des imidazoles monosubstitués de formule II 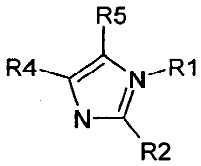 où
R1 représente un radical organique comprenant 1 à 20 atomes de carbone,
R2, R4, et R5 représentent, indépendamment l'un de l'autre, un atome d'hydrogène ou un radical organique comprenant 1 à 20 atomes de carbone.

7. Procédé selon l'une quelconque des revendications 1 à 6, **caractérisé en ce que** R1 dans les formules I et II et R3 dans la formule I représentent, indépendamment l'un de l'autre, un groupe C₁-C₁₀-alkyle.

8. Procédé selon l'une quelconque des revendications 1 à 7, **caractérisé en ce que** R2, R4 et R5 représentent, indépendamment l'un de l'autre, un atome d'hydrogène ou un groupe C₁-C₁₀-alkyle.

9. Procédé selon l'une quelconque des revendications 1 à 8, **caractérisé en ce que** X représente l'anion d'un composé présentant au moins un groupe carboxylate.

10. Procédé selon l'une quelconque des revendications 1 à 9, **caractérisé en ce que** X représente l'anion acétate.

11. Procédé selon l'une quelconque des revendications 1 à 10, **caractérisé en ce que** les mélanges contiennent de l'eau, un solvant miscible à l'eau ou leurs mélanges comme solvant.

12. Procédé selon l'une quelconque des revendications 1 à 11, **caractérisé en ce qu'**il s'agit, pour l'oxydant, d'oxygène, de peroxydes ou de peracides.

13. Procédé selon l'une quelconque des revendications 1 à 12, **caractérisé en ce qu'**il s'agit, pour l'oxydant, de peroxyde d'hydrogène.

14. Procédé selon l'une quelconque des revendications 1 à 13, **caractérisé en ce que** l'oxydation est réalisée à 20 jusqu'à 10°C.

15. Procédé selon l'une quelconque des revendications 1 à 14, **caractérisé en ce que** l'oxydant est ajouté avant l'élimination du solvant.

16. Mélanges, pouvant être obtenus par un procédé selon l'une quelconque des revendications 1 à 15.

17. Utilisation des mélanges selon la revendication 16 comme solvant.

18. Utilisation des mélanges selon la revendication 16 comme solvant pour la cellulose.
